# EUROPEAN PATENT SPECIFICATION

(11) **EP 2 734 254 B1**
(45) Date of publication and mention of the grant of the patent: **14.06.2017**
(21) Application number: 12740831.8
(22) Date of filing: 19.07.2012
(51) Int. Cl.: A61M 5/19, A61M 5/315, A61M 5/31

(54) **UNIDIRECTIONAL PLUNGER DEVICE FOR SYRINGE**
UNIDIREKTIONALE KOLBENVORRICHTUNG FÜR EINE SPRITZE
DISPOSITIF DE PLONGEUR UNIDIRECTIONNEL POUR SERINGUE

(30) Priority: 21.07.2011 US 201113187978
(43) Date of publication of application: 28.05.2014
(73) Proprietor: Howmedica Osteonics Corp., Mahwah, NJ 07430 (US)
(72) Inventor: MADDEN, Kevin, Newcastle West Limerick (IE); HENRY, John, Castletroy Limerick (IE); LEGNER, Andreas, 35796 Weinbach (DE); ROISIN, Nolan, Rochestown, County Cork (IE)
(74) Representative: Liebetanz, Michael
(86) International application number: PCT/US2012/047343
(87) International publication number: WO 2013/013011

(56) References cited:
- WO-A1-94/03392
- FR-A- 1 054 173
- US-A- 4 946 441
- US-A1- 2004 122 361
- US-B1- 6 296 625

## Description

### BACKGROUND OF THE INVENTION

The present invention relates to syringes and to apparatus for resisting proximal movement of plungers of such syringes.

Conventionally, syringes, such as syringes for dispensing bone cement, may be provided to a user in a prefilled state. For example, syringes for dispensing two-part curable bone cement compositions may have two barrels for separately containing components of those compositions that will cure upon being combined. The body of such a multi-barrel syringe may be provided to the user with the components of the bone cement already located in the syringe barrels.

Before providing a prefilled syringe to the user, the syringe may undergo several processing steps. For example, a sealing system may be attached to the distal end of the syringe body, and the prefilled body may be sterilized (e.g., by exposure to gamma radiation). The syringe body and other components of the syringe system may also be secured into a specially designed tray or package, which integrated package is shipped to the user.

In order to dispense the bone cement composition from the syringe system, the user may first need to assemble portions of the system. For example, the user may detach the sealing system from the distal end of the syringe body and attach a static mixer in
Closest prior art is US 4,946,441 which describes a hypodermic syringe that can be filled with liquid from a container. It describes a syringe system with a syringe body and a plunger in which the syringe body is adapted so that the syringe body can be manipulated to push the plunger towards the distal end, but the syringe body cannot be manipulated to move the plunger towards the proximal end, and the position of the plunger in the syringe body can be adjusted at any of a plurality of positions.

Another relevant document is patent WO 94/03392 which describes a variable proportion dispenser. It describes two one-way drivers and threaded dosage adjusters that are mounted in the sliding body of the dispenser. These drive systems allow a range of dose selections. The manipulator therefore can control the amount delivered during a delivery stroke. A cartridge release assembly allows to release the drive stems of the one-way drivers permitting the drive stems to be returned to their original positions.

Although effort has been devoted in the art heretofore to optimization of such syringe systems and their packaging, still further improvement would be desirable.

### BRIEF SUMMARY OF THE INVENTION

One aspect of the present invention provides an apparatus for resisting proximal movement of a plunger of a syringe, which syringe includes a body having a proximal end and a distal end. The apparatus according to this aspect of the invention desirably includes a base member and a ratchet mechanism connected to the base member. The base member is preferably adapted to securely attach to an exterior surface of the body of the syringe. The ratchet mechanism is preferably structured and arranged to contact the plunger so as to permit distal movement of the plunger with respect to the body but resist proximal movement of the plunger with respect to the body.

According to one aspect of the invention, the base member is shaped to securely attach to the proximal end of the body. In accordance with this aspect of the invention, a shape of the base member along a plane perpendicular to a longitudinal axis of the syringe body substantially matches a shape of the syringe body along that plane.

According to another aspect of the invention, the base member includes a passage therethrough, which passage is adapted to receive a portion of the plunger therethrough, and which passage is aligned with an opening at the proximal end of the syringe through which a portion of the plunger is received. In accordance with this aspect of the invention, the passage through the base member preferably has substantially the same geometry as the opening at the proximal end of the syringe body. In another alternative, the proximal end of the syringe body may have multiple openings, the
plunger may have an associated plurality of plunger rods, and the base member may include a plurality of passages therethrough, the passages being aligned with the respective openings and adapted to receive the respective plunger rods therethrough.

According to yet another aspect of the invention, the apparatus may further comprise a clip connected to the base member. The clip, in accordance with this aspect of the invention, is desirably structured and arranged to securely attach to a feature on the exterior surface of the syringe body. According to this aspect of the invention, that feature may include a portion of a gripping member extending away from a longitudinal axis of the syringe body.

In accordance with yet a further aspect of the invention, the ratchet mechanism includes a pawl adapted to engage a plunger rod of the plunger. According to this aspect of the invention, the pawl is adapted to engage a plurality of teeth on the plunger rod.

According to yet further aspects of the invention, the base member may include at least one projection adapted to engage a plunger rod of the plunger. In accordance with this aspect of the invention, the plunger stabilizes an orientation of the plunger rod with respect to the syringe body. Also in accordance with this aspect of the invention, the projection may extend into the passage of the base member.

In accordance with additional aspects of the invention, the base member is detachable from the exterior surface of the syringe body.

Further aspects of the invention provide a syringe. A syringe in accordance with such an aspect of the invention includes a body, a plunger, and an apparatus in accordance with any of the above aspects of the invention. The body of such a syringe preferably has a proximal end, a distal end, and an exterior surface. The body also desirably includes at least one barrel, each of which defines a fluid channel. The plunger of the syringe according to this aspect of the invention preferably has at least one plunger rod being slidably receivable at least partially within a respective fluid channel. The base member of the apparatus in accordance with this aspect of the invention desirably is securely attached to the exterior surface of the body.

Yet further aspects of the invention provide a method for modifying a syringe. The method according to this aspect of the invention desirably includes securely attaching the base member of an apparatus in accordance with any of the above aspects of the invention to an exterior surface of a body of a syringe. The base member is preferably attached to the exterior surface such that the ratchet mechanism of the apparatus contacts a plunger of the syringe. Desirably, the contact between the ratchet mechanism and the plunger is such that distal movement of the plunger with respect to the body is permitted, but proximal movement of the plunger with respect to the body is resisted.

### BRIEF DESCRIPTION OF THE DRAWINGS

FIG. 1 is a perspective view of a portion of a syringe system in accordance with one embodiment of the invention.
FIG. 2A is a perspective view of the syringe body of the portion of the syringe system illustrated in FIG. 1.
FIG. 2B is a side view of the syringe body of FIG. 2A.
FIG. 2C is a rear view of the syringe body of FIG. 2A.
FIG. 3 is a perspective view of a portion of a syringe system including a unidirectional plunger device in accordance with one embodiment of the invention.
FIGS. 4A and 4B are perspective views of the unidirectional plunger device of FIG. 3.
FIG. 5A is a perspective view of the syringe body and the unidirectional plunger device of the portion of the syringe system illustrated in FIG. 3.
FIG. 5B is a side view of the syringe body and unidirectional plunger device of FIG. 5A.
FIG. 5C is a rear view of the syringe body and unidirectional plunger device of FIG. 5A.
FIG. 6A is a perspective view of the plunger of the portion of the syringe system illustrated in FIG. 3.
FIG. 6B is a side view of the plunger of FIG. 6A.
FIG. 7 is a perspective view of a portion of the portion of the syringe system including the unidirectional plunger device illustrated in FIG. 3.
FIG. 8A is a perspective view of a portion of the plunger illustrated in FIG. 6A.
FIG. 8B is a perspective view of a portion of the unidirectional plunger device illustrated in FIGS. 4A-B.

### DETAILED DESCRIPTION

As used herein, the term "distal" relates to the direction away from the operator of the syringe during use, while the term "proximal" relates to the direction towards the operator.

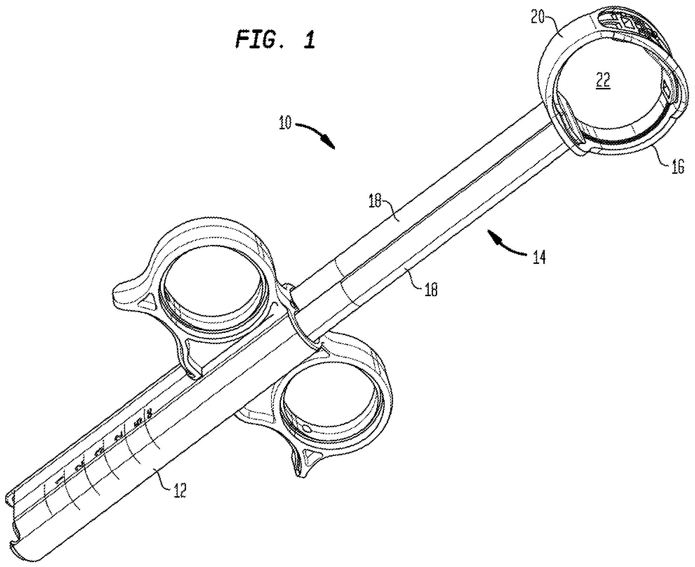

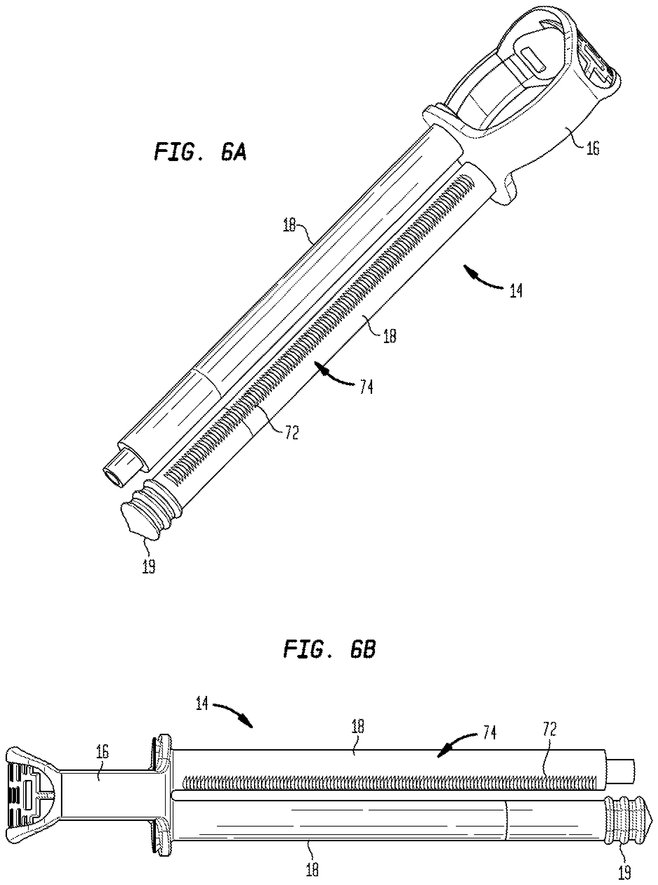

FIG. 1 illustrates a portion of a syringe system 10, including a syringe body 12 and a plunger 14. The plunger includes a grip element 16 and two plunger rods 18, which may all be integrally formed together (e.g., from a polymer material, such as polyamide). The plunger may also include a plunger tip 19 (*see* FIGS. 6A-B) located at the distal end of each plunger rod 18 and formed from an elastomeric material such as silicone rubber. The grip element 16 may include a loop 20 defining an opening 22 dimensioned to receive at least one operator's finger. Loop 20 allows an operator to grab plunger 14 and push or pull it with respect to body 12. Each of the plunger rods 18 may have a substantially cylindrical shape.

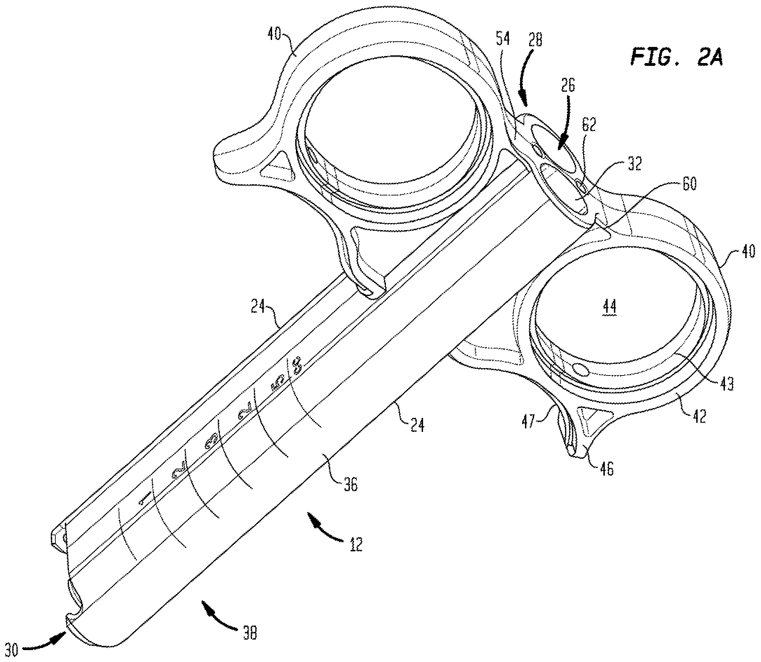

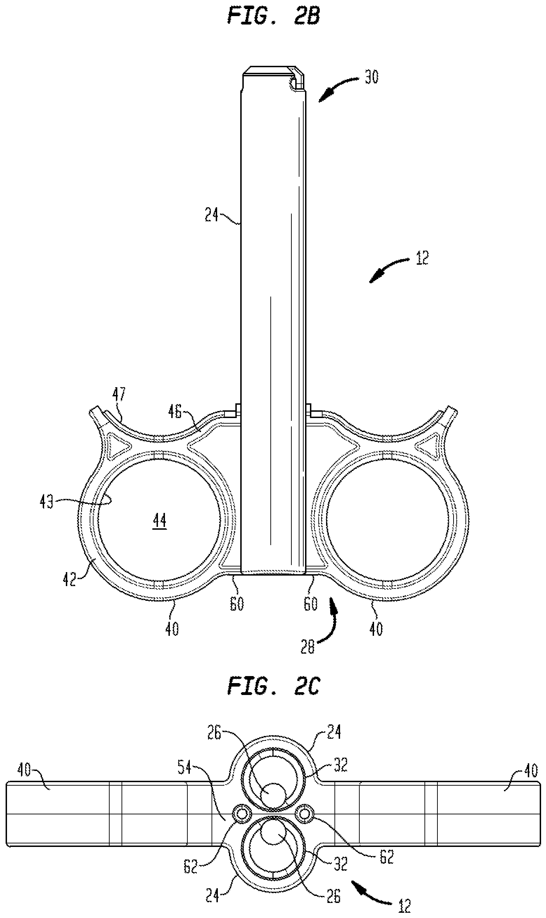

As shown in FIGS. 2A-C, the body 12 has two barrels 24, each defining a substantially cylindrical channel 26 extending through the body 12 between its proximal end 28 and its distal end 30. The channels 26 are configured to separately hold different components of a multi-part curable composition to be dispensed by the syringe system 10, such as two-part calcium phosphate cement or two-part epoxy resin. The channels 26 slidably receive the respective plunger rods 18 therein, with each of the plunger tips 19 sealingly engaging the inner periphery of the respective barrel 24, such that distal movement of the plunger 14 displaces the components of the curable composition and ejects them from the distal end 30 of the body 12. The channels 26 terminate in openings 32 at the proximal end 28 of the syringe body 12 through which the plunger rods 18 pass. A septum or internal wall 34 separates the channels 26 and prevents the separately contained fluids from mixing together.

The distal end 30 of the body 12 may be configured to connect to other components, such as a sealing system and a static mixer. An outer surface 36 of the body 12 may include markers or indicia 38 for measuring the volume of the components contained in the channels 26. The body 12 may also include gripping members 40 positioned on substantially opposite sides of the body 12 and extending radially outwardly from the outer surface 36. Each gripping member 40 may include a loop 42 defining an aperture or hole 44 and an undulating bar or element 46. The hole 44 and undulating bar 46 may each be dimensioned to receive at least one operator's finger.

The body 12 is preferably transparent and may be formed from materials such as cyclic olefin copolymer or glass. An overmolded material (not shown) may also cover all or a portion of the body 12. For example, a thermoplastic elastomer may be overmolded onto the inner surface 43 of each loop 42 and/or onto the distal surface 47 of each undulating bar 46. Such an overmolded material, which may or may not define a textured surface, may increase the grip between the operator's fingers and the syringe body 12.

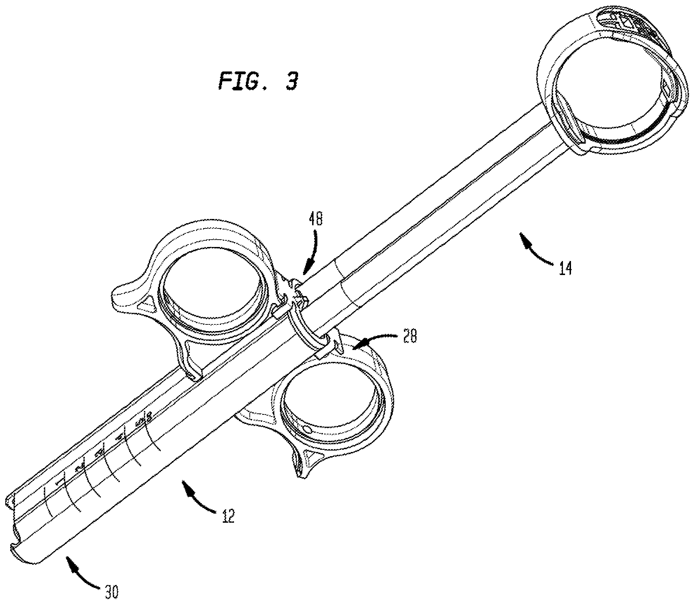

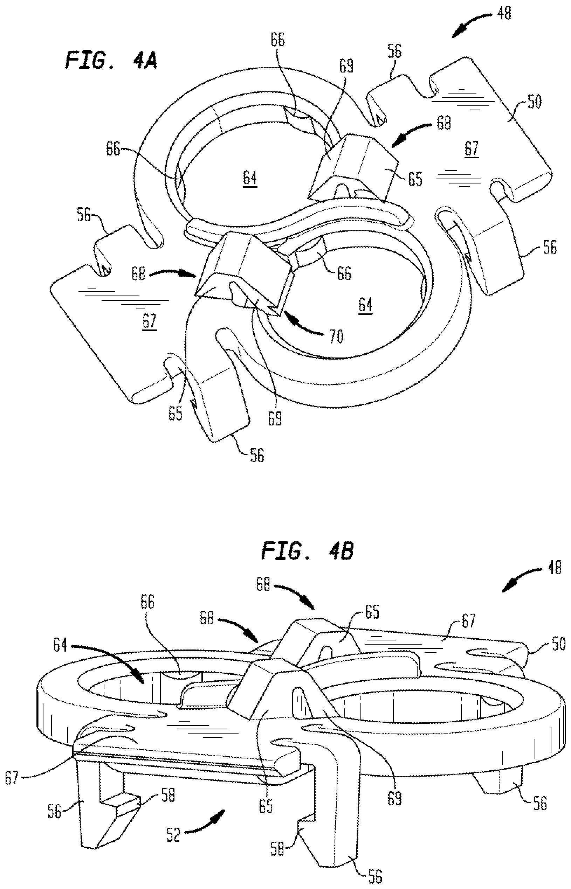

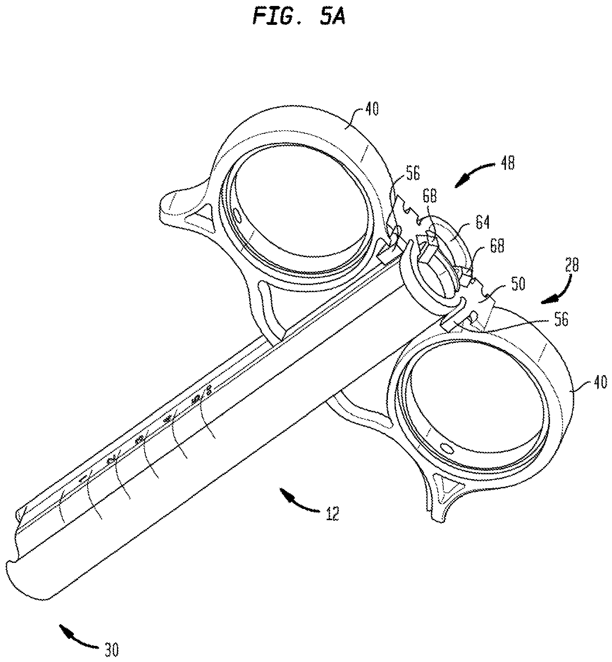

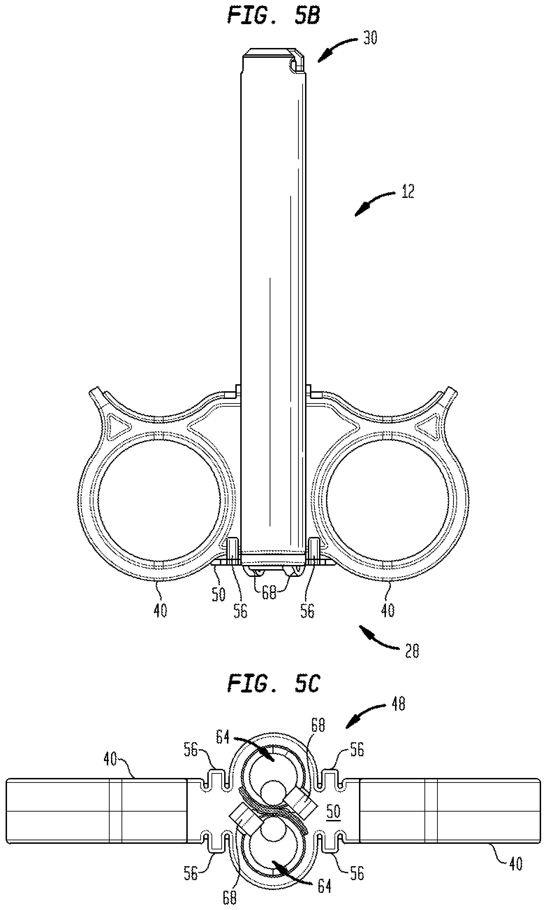

FIG. 3 illustrates the portion of the syringe system 10 illustrated in FIG. 1 in which a unidirectional plunger device 48 is attached to the proximal end 28 of the syringe body 12. Views of the unidirectional plunger device 48 isolated from the syringe body 12 are illustrated in FIGS. 4A and 4B. Various views of the unidirectional plunger device 48 attached to the proximal end 28 of the syringe body 12 are illustrated in FIGS. 5A-C.

The unidirectional plunger device 48 includes a base member 50, which may be in the form of a generally planar component that generally matches the shape of at least a portion of the syringe body 12 at the proximal end 28. The unidirectional plunger device 48 is configured to be securely attached to the proximal end 28 of the body 12, with the underside 52 of the base member 50 abutting the proximal end surface 54 of the body 12 in the region adjacent the openings 32 (see FIGS. 2A-C). The unidirectional plunger device 48 may be attached to the proximal end 28 of the body 12 by way of a clip comprising multiple (e.g., four) opposing, flexible snap latches 56 extending distally from the base member 50. Each snap latch 56 may include a bump 58 at its distal end for interlocking with a lip 60 (see FIGS. 2A-C) at the proximal end 28 of the body 12, so as to secure the unidirectional plunger device 48 to the proximal end 28. The lip 60 may be a portion of a gripping member 40.

The snap latches 56 are preferably designed so as to securely affix the unidirectional plunger device 48 to the proximal end 28 of the syringe body 12, although the snap latches 56 may also allow the unidirectional plunger device 48 to be detached from the body 12 (if desired) upon manipulation of the snap latches 56 by a user. The underside 52 of the base member 50 may also include one or more distally extending projections (not shown) configured to be received by corresponding recesses 62 (see FIGS. 2A-C) in the proximal end 28 of the body 12, in order to provide further stability for the unidirectional plunger device 48.

The unidirectional plunger device 48 may include two passages 64 through the base member 50, which passages 64 are aligned with the respective openings 32 in the proximal end 28 of the body 12, such that the plunger rods 18 pass through the passages 64. The passages 64 may each have a generally circular cross-sectional shape that substantially matches the size and shape of the openings 32. The unidirectional plunger device 48 may also include one or more (e.g., three) projections 66 spaced around each of the passages 64 in the base member 50. Each of the projections 66 is desirably configured to engage one of the plunger rods 18 so as to provide stability to that plunger rod 18, and, in turn, the entire plunger 14. That is, the combined engagement of all of the projections 66 against a particular plunger rod 18 preferably constrains the lateral position of that plunger rod 18 within the passage 64, which desirably reduces wobbling and maintains the plunger rod 18 in an axial alignment within the respective channel 26.

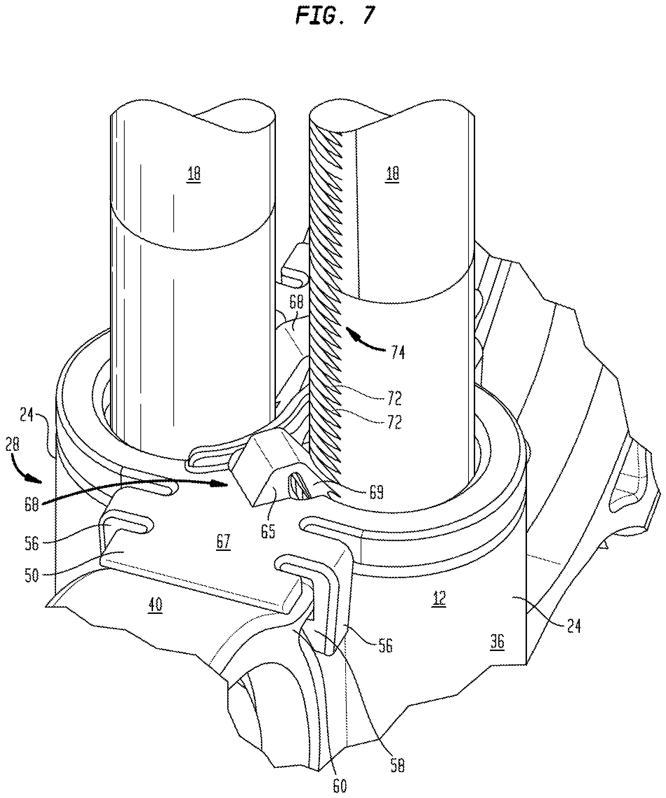

The unidirectional plunger device 48 may also include a ratchet mechanism configured to engage at least one of the plunger rods 18 when the unidirectional plunger device 48 is attached to the proximal end 28 of the syringe body 12. In one embodiment, the ratchet mechanism may include two pawls 68, each located along the periphery of a respective passage 64. Each pawl 68 includes a base 65 projecting proximally from the top surface 67 of the base member 50 and an arm 69 extending inward from the base 65 towards the center of the passage 64. The arms 69 are preferably flexible, such that the arms 69 can pivot with respect to the base 65. Each arm has a free end 70 configured to engage a respective plunger rod 18. Each arm 69 extends from the base 65 towards the respective plunger rod 18 in the distal direction and at an acute angle with respect to the longitudinal axis of the plunger rod 18. The height of the base 65 and the configuration of the arm 69 may be such that the free end 70 of the arm does not extend distally of the underside 52 of the base member 50. The free ends 70 of the arms 69 are preferably shaped to engage teeth 72 (*see* FIGS. 6A-B) provided along the plunger rods 18. As shown in FIGS. 6A-B, the teeth 72 may be arranged in a linear series 74 extending along all or a portion of each of the plunger rods 18. FIG. 7 illustrates the engagement between the pawls 68 and the teeth 72.

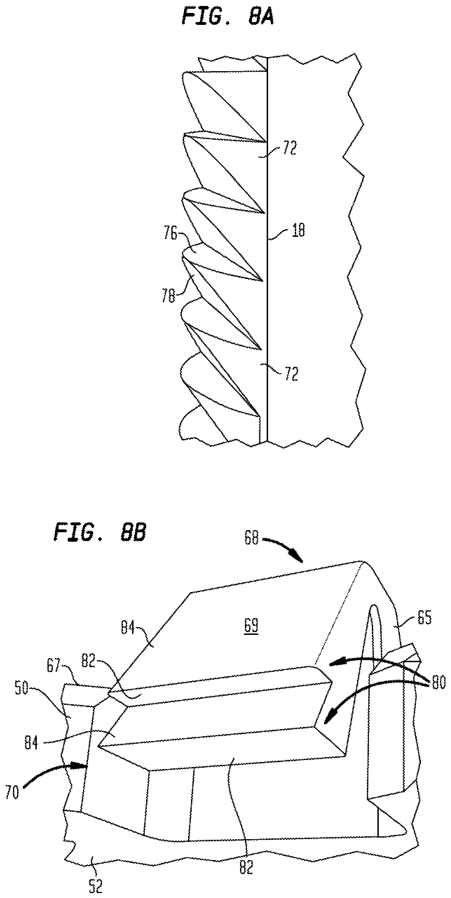

As illustrated in FIG. 8A, the teeth 72 may each include a proximally oriented face 76 and a distally oriented face 78. The proximally oriented face 76 may be oriented substantially perpendicular to the longitudinal axis of the respective plunger rod 18, and the distally oriented face 78 may define an acute angle with respect to that longitudinal axis. As illustrated in FIG. 8B, the free end 70 of each pawl 68 may be shaped so as to define one or more (e.g., two) tooth engagement portions 80. Each tooth engagement portion 80 may include a distally oriented face 82 and a proximally oriented face 84. The distally oriented face 82 may be oriented substantially perpendicular to the longitudinal axis of the respective plunger rod 18, and the proximally oriented face 84 may define an acute angle with respect to that longitudinal axis.

Preferably the engagement between the pawls 68 and the teeth 72 is such that the plunger 14 is permitted to move distally with respect to the body 12, while proximal movement of the plunger 14 is resisted. In this regard, the engagement between the distally oriented faces 82 of the tooth engagement portions 80 of the pawls 68 and the proximally oriented faces 76 of teeth 72 resists proximal movement of the plunger 14. However, when the user pushes the plunger 14 distally, the engagement between the angled distally oriented faces 78 of the teeth 72 and the angled proximally oriented faces 84 of the pawls 68 causes the arms 69 of the pawls 68 to pivot distally, such that their free ends 70 move away from the longitudinal axes of the respective plunger rods 18 and permit the plunger rods 18 to move distally.

Although the unidirectional plunger device resists proximal movement of the plunger 14, this feature may be bypassed by a user if desired. In one example, by twisting the plunger 14 illustrated in FIGS. 3 and 7 in a counterclockwise direction while pulling in the proximal direction, each series 74 of teeth 72 may be moved out of engagement with the respective pawl 68, thereby allowing the user to more easily move the plunger 14 proximally. If the material of the plunger 14 is sufficiently flexible, this bypassing may occur due to the twisting of the plunger 14 causing each of the plunger rods 18 to rotate slightly counterclockwise until the engagement between the pawls 68 and the teeth 72 is reduced or eliminated.

Some or all components of the unidirectional plunger device 48 (*e.g.,* the base member 50, the snap latches 56, and the pawls 68) may be formed as separate components that are subsequently connected together or they may be integrally formed together as a unit. For example, the entire unidirectional plunger device 48 may be integrally molded from a polymer material, such as polyamide.

Among the benefits believed to be provided by the present invention is the prevention of undesirable proximal movement of the plunger 14. For example, during sterilization of the prefilled syringe body 12 (*e.g*., by exposure to gamma radiation), the components of a multi-part curable composition may expand, which might result in the plunger 14 being pushed proximally out of the proximal end 28 of the body 12. The plunger 14 may also move proximally during shipment (*e.g*., by expansion of the curable composition components when exposed to heat). Desirably, the unidirectional plunger device reduces or eliminates such unwanted proximal plunger movement without significantly impacting the movement of the plunger in the distal direction. Additionally, the unidirectional plunger device preferably provides the beneficial resistance to proximal plunger movement at various plunger positions (corresponding to various fluid levels in the channels), at least due to the continuous engagement between the device and the plunger rods as the position of the plunger within the channels varies. The inclusion of projections 66 on the device also desirably provides stability to the plunger rods 18 (as discussed above) during shipping, assembly, and injection.

The unidirectional plunger device 48 may be provided as a retrofit to an existing syringe design. For example, the base member 50 can be shaped so that it matches the shape of the proximal end 28 of one or more existing syringe designs. Similarly, the passages 64 through the base member 50 are preferably configured with the same geometry as the openings 32 in the proximal end 28 of one or more such existing syringe bodies 12. Desirably the unidirectional plunger device 48 does not interfere with the operation of the existing syringe design. In this regard, the device 48 preferably attaches to an exterior surface of the syringe body 12, such as by abutting the proximal end surface 54 of the body 12 and clipping onto a feature (e.g., lip 60) on the body 12, as discussed above. In this way, the device 48 preferably does not extend into or otherwise interfere with the interior of the syringe body 12.

Many variations of the above described embodiments are possible within the scope of the present invention. For example, the present invention is not limited to two-barrel syringes. For instance, a unidirectional plunger device in accordance with the present invention may be used in conjunction with a syringe having a single barrel or three or more barrels. In such cases, the associated components can be adjusted accordingly (*e.g.,* by providing the appropriate number of plunger rods 18, passages 64 through the base member 50, associated pawls 68, etc.).

In other variations, the plunger rods 18 need not include teeth 72, and the ratchet mechanism may be configured to engage one or more of the plunger rods 18 so as to provide the desired unidirectional movement. For example, the free ends 70 of the pawl arms 69 may have a very sharp tip. Due to the angled, distally extending orientation of the arms 69, the tip can be arranged to slide along the smooth outer surface of the associated plunger rod 18 when the plunger 14 is moving in the distal direction, while the sharp tip can dig into and resist movement of the plunger rod 18 in the proximal direction. In such an embodiment, all or a portion of the ratchet mechanism may be constructed of a material which is substantially harder than the material of the plunger rods 18. For example, the sharp tip may be constructed of a metallic material. The above-described "toothless" embodiment may be beneficial, particularly in the case of a retrofit, as the existing plunger may not need to be modified or replaced so as to include teeth 72.

In yet other variations, the unidirectional plunger device 48 need not be removable from the syringe body 12. For example, an adhesive can be used to affix the device to the body 12, or the mechanical fixation device (*e.g*., snap latches) could be designed to result in permanent affixation.

Based on the design of the syringe with which the unidirectional plunger device is used, the device may extend into or be entirely located within the channels of the syringe body. For example, if the design of the syringe system is such that the cross-sectional area of the plunger rods is substantially smaller than that of the barrels, there may be sufficient space between the plunger rods and the inner periphery of the barrels to locate an embodiment of a unidirectional plunger device within the channels.

Although not shown herein, it is noted that plungers in accordance with embodiments of the present invention may include connectors located between the plunger rods and the plunger tips.

Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the scope of the present invention as defined by the appended claims.

## Claims

1. An apparatus for resisting proximal movement of a plunger (14) of a syringe (10), the syringe including a body (12) having a proximal end (28) and a distal end (30), the apparatus comprising:
a base member (50) adapted to securely attach to an exterior surface (54) of the body (12) of the syringe (10); and
a ratchet mechanism (68) connected to the base member (50), the ratchet mechanism being structured and arranged to contact a plunger rod (18) of the plunger (14) when the plunger is located at a plurality of positions along the body (12) of the syringe (10), such that, at any one of the plurality of positions of the plunger (14), the contact between the ratchet mechanism (68) and the plunger rod (18) permits distal movement of the plunger (14) with respect to the body (12) but resists proximal movement of the plunger (14) with respect to the body;
**characterized in that**:
the base member (50) comprises a generally planar component that generally matches a shape of at least a portion of the body (12) at the proximal end (28)a.

2. The apparatus of claim 1, wherein the plunger rod (18) has a substantially cylindrical shape, and wherein the base member (50) includes at least one projection (66) adapted to engage the plunger rod (18) of the plunger (14) when the base member (50) is attached to the body (12), the projection (66) stabilizing an orientation of the plunger rod (18) with respect to the syringe body (12).

3. The apparatus of claim 2, wherein the proximal end (28) of the syringe body (12) has an opening (32) adapted to receive the plunger rod (18) therethrough, and wherein the base member (50) includes a passage (64) therethrough adapted to receive the plunger rod (18) therethrough, the passage being adapted to align with the opening (32) at the proximal end (28) of the syringe body (12) when the base member (50) is attached to the body, and wherein the projection (66) extends into the passage (64) of the base member (50).

4. The apparatus of any one of claims 1 to 3, wherein the base member (50) is shaped to securely attach to the proximal end (28) of the body (12) such that a shape of the base member along a plane perpendicular to a longitudinal axis of the syringe body (12) substantially matches a shape of the syringe body along the plane.

5. The apparatus of any one of claims 1 to 4, wherein the proximal end (28) of the syringe body (12) has an opening (32) adapted to receive a portion of the plunger (14) therethrough, and wherein the base member (50) includes a passage (64) therethrough adapted to receive the portion of the plunger (14) therethrough, the passage (64) being adapted to align with the opening (32) at the proximal end (28) of the syringe body (12) when the base member (50)is attached to the body.

6. The apparatus of any one of claims 1 to 5, further comprising a clip (56) connected to the base member (50), the clip being structured and arranged to securely attach to a feature (40) on the exterior surface (54) of the syringe body (12).

7. The apparatus of claim 6, wherein the feature (40) includes a portion of a gripping member (40) extending away from a longitudinal axis of the syringe body (12).

8. The apparatus of any one of claims 1 to 7, wherein the ratchet mechanism includes a pawl (68) adapted to engage a plurality of teeth (72) on the plunger rod (18) of the plunger (14) when the base member (50) is attached to the body (12).

9. A syringe (10), comprising:
a body (12) having a proximal end (28), a distal end (30), and an exterior surface (54), the body including at least one barrel (24), each of the at least one barrel defining a fluid channel (26);
a plunger (14) having at least one plunger rod (18), each of the at least one plunger rod (18) being slidably receivable at least partially within a respective one of the fluid channels (26); and
an apparatus as recited in any one of claims 1 to 8, the base member (50) of the apparatus being securely attached to the exterior surface (54) of the body (12).

10. The syringe (10) of claim 9, wherein the base member (50) is securely attached to the proximal end (28) of the body (12).

11. The syringe (10) of claims 9 or 10, wherein the base member (50) includes at least one passage (64) therethrough, each of the at least one passage being adapted to receive a respective one of the at least one plunger rod (18) therethrough, and wherein the base member (50) includes a plurality of projections (66) extending into at least one of the passages (64) and engaging at least one of the plunger rods (18) to stabilize an orientation of the at least one of the plunger rods (18) with respect to the syringe body (12).

12. The syringe (10) of any one of claims 9 to 11, wherein the body (12) includes at least one feature (40) on the exterior surface (54) thereof, and wherein the apparatus includes a clip (56) connected to the base member (50), the clip being securely attached to the feature (40).

13. The syringe (10) of any one of claims 9 to 12, wherein the base member (50) is detachably attached to the exterior surface (54) of the body (12).

14. The syringe (10) of any one of claims 9 to 13, wherein at least one of the plunger rods (18) includes a plurality of teeth (72) at least partially therealong, and wherein the ratchet mechanism of the apparatus includes a pawl (68) adapted to engage the teeth (72).

15. A method for modifying a syringe (10), comprising:
securely attaching the base member (50) of an apparatus as recited in any one of claims 1 to 8 to an exterior surface (54) of a body (12) of a syringe (10), such that the ratchet mechanism (68) of the apparatus contacts a plunger (14) of the syringe so as to permit distal movement of the plunger (14) with respect to the body (12) but resist proximal movement of the plunger (14) with respect to the body.

## Patentansprüche

1. Vorrichtung zum Widerstehen einer proximalen Bewegung eines Kolbens (14) einer Spritze (10), wobei die Spritze einen Körper (12) mit einem proximalen Ende (28) und einem distalen Ende (30) aufweist, wobei die Vorrichtung aufweist:
- ein Basiselement (50), das geeignet ist, sicher an einer äusseren Oberfläche (54) des Körpers (12) der Spritze (10) befestigt zu sein; und
- einen Ratschenmechanismus (68), der mit dem Basiselement (50) verbunden ist, wobei der Ratschenmechanismus so ausgestaltet und angeordnet ist, dass er eine Kolbenstange (18) des Kolbens (14) berührt, wenn der Kolben an einer Vielzahl von Positionen entlang des Körpers (12) der Spritze (10) angeordnet ist, so dass, an einer jeden der Vielzahl von Positionen des Kolbens (14), der Kontakt zwischen dem Ratschenmechanismus (68) und der Kolbenstange (18) eine distale Bewegung des Kolbens (14) in Bezug auf den Körper (12) zu ermöglichen, aber einer proximalen Bewegung des Kolbens (14) in Bezug auf den Körper zu widerstehen;
**dadurch gekennzeichnet, dass** das Basiselement (50) eine im Wesentlichen planare Komponente umfasst, die im Allgemeinen mit einer Form von mindestens einem Abschnitt des Körpers (12) an dem proximalen Ende (28) übereinstimmt.

2. Vorrichtung nach Anspruch 1, wobei die Kolbenstange (18) eine im wesentlichen zylindrische Form hat, und wobei das Basiselement (50) mindestens einen Vorsprung (66) umfasst, der ausgebildet ist, um in die Kolbenstange (18) des Kolbens (14) einzugreifen, wenn das Basiselement (50) an dem Körper (12) befestigt ist, wobei der Vorsprung (66) eine Orientierung der Kolbenstange (18) in Bezug auf den Spritzenkörper (12) stabilisiert.

3. Vorrichtung nach Anspruch 2, wobei das proximale Ende (28) des Spritzenkörpers (12) eine Öffnung (32) aufweist, die so ausgebildet ist, dass sie die Kolbenstange (18) durch sie hindurch aufnimmt, und wobei das Basiselement (50) einen Durchgang (64) durch es hindurch aufweist, der so ausgebildet ist, dass er die Kolbenstange (18) durch ihn hindurch aufnimmt, wobei der Durchgang so ausgebildet ist, dass er sich mit der Öffnung (32) des proximalen Endes (28) des Spritzenkörpers (12) ausrichtet, wenn das Basiselement (50) an dem Körper befestigt ist, und wobei sich der Vorsprung (66) in den Durchgang (64) des Basiselements (50) erstreckt.

4. Vorrichtung nach irgendeinem der Ansprüche 1 bis 3, wobei das Basiselement (50) so ausgeformt ist, um sicher an dem proximalen Ende (28) des Körpers (12) befestigt zu werden, so dass eine Form des Basiselements entlang einer Ebene senkrecht zu einer Längsachse des Spritzenkörpers (12) im Wesentlichen einer Form des Spritzenkörpers entlang der Ebene entspricht.

5. Vorrichtung nach irgendeinem der Ansprüche 1 bis 4, wobei das proximale Ende (28) des Spritzenkörpers (12) eine Öffnung (32) aufweist, die ausgestaltet ist, um einen Abschnitt des Kolbens (14) durch sie hindurch aufzunehmen, und wobei das Basiselement (50) einen Durchgang (64) durch es hindurch aufweist, die ausgestaltet ist, um einen Abschnitt des Kolbens (14) durch ihn hindurch aufzunehmen, auf, wobei der Durchgang (64) so ausgestaltet ist, dass er mit einer Öffnung (32) an dem proximalen Ende (28) des Spritzenkörpers (12) ausgerichtet ist, wenn das Basiselement (50) an dem Körper befestigt ist.

6. Vorrichtung nach irgendeinem der Ansprüche 1 bis 5, ferner eine Klammer (56) umfassend, die mit dem Basiselement (50) verbunden ist, wobei die Klammer so ausgestaltet und angeordnet ist, dass sie sicher an einem Merkmal (40) an der Aussenfläche (54) des Spritzenkörpers (12) befestigt ist.

7. Vorrichtung nach Anspruch 6, wobei das Merkmal (40) einen Abschnitt eines Greifelements (40) umfasst, das sich von einer Längsachse des Spritzenkörpers (12) weg erstreckt.

8. Vorrichtung nach irgendeinem der Ansprüche 1 bis 7, wobei der Ratschenmechanismus eine Klinke (68) aufweist, die so ausgebildet ist, dass sie mit einer Vielzahl von Zähnen (72) auf der Kolbenstange (18) des Kolbens in Eingriff kommt, wenn das Basiselement (50) an dem Körper (12) befestigt ist.

9. Spritze (10), umfassend:
- einen Körper (12) mit einem proximalen Ende (28), einem distalen Ende (30) und einer äusseren Oberfläche (54), wobei der Körper mindestens einen Zylinder (24) umfasst, wobei jeder des mindestens einen Zylinders einen Fluidkanal (26) definiert;
- einen Kolben (14) mit mindestens einer Kolbenstange (18), wobei jede der mindestens einen Kolbenstange (18) zumindest teilweise innerhalb eines jeweiligen der Fluidkanäle (26) gleitend verschiebbar aufnehmbar ist; und
- eine Vorrichtung nach irgendeinem der Ansprüche 1 bis 8, wobei das Basiselement (50) der Vorrichtung sicher an der Aussenfläche (54) des Körpers (12) befestigt ist.

10. Spritze (10) nach Anspruch 9, wobei das Basiselement (50) sicher am proximalen Ende (28) des Körpers (12) befestigt ist.

11. Spritze (10) nach Anspruch 9 oder 10, wobei das Basiselement (50) mindestens einen Durchgang (64) durch es hindurch aufweist, wobei jeder des mindestens einen Durchgangs so ausgebildet ist, dass er jeweils eine der mindestens einen Kolbenstange (18) durch ihn hindurch aufnehmen kann, und wobei das Basiselement (50) eine Vielzahl von Vorsprüngen (66) umfasst, die sich in mindestens einen der Durchgänge (64) hinein erstrecken und mit mindestens einer der Kolbenstangen (18) in Eingriff stehen, um eine Orientierung der mindestens einen der Kolbenstangen (18) in Bezug auf den Spritzenkörper (12) zu stabilisieren.

12. Spritze (10) nach irgendeinem der Ansprüche 9 bis 11, wobei der Körper (12) mindestens ein Merkmal (40) auf seiner Aussenfläche (54) aufweist, und wobei die Vorrichtung eine Klammer (56) aufweist, die mit dem Basiselement (50) verbunden ist, wobei die Klammer sicher an dem Merkmal (40) befestigt ist.

13. Spritze (10) nach irgendeinem der Ansprüche 9 bis 12, wobei das Basiselement (50) in lösbarer Weise an der Aussenfläche (54) des Körpers (12) angebracht ist.

14. Spritze (10) nach irgendeinem der Ansprüche 9 bis 13, wobei mindestens eine der Kolbenstangen (18) eine Vielzahl von Zähnen (72) zumindest teilweise entlang derselben aufweist, und wobei der Ratschenmechanismus der Vorrichtung eine Klinke (68) aufweist, die so ausgebildet ist, um mit den Zähnen (72) in Eingriff zu gelangen.

15. Verfahren zum Modifizieren einer Spritze (10), umfassend:
- ein sicheres Befestigen des Basiselementes (50) einer Vorrichtung nach irgendeinem der Ansprüche 1 bis 8 an einer Aussenfläche (54) eines Körpers (12) einer Spritze (10), so dass der Ratschenmechanismus (68) der Vorrichtung einen Kolben (14) der Spritze so berührt, um eine distale Bewegung des Kolbens (14) in Bezug auf den Körper (12) zu gestatten, aber einer proximalen Bewegung des Kolbens (14) in Bezug auf den Körper zu widerstehen.

## Revendications

1. Appareil pour résister au mouvement proximal d'un piston (14) d'une seringue (10), la seringue comprenant un corps (12) ayant une extrémité proximale (28) et une extrémité distale (30), où l'appareil comprend:
- un élément de base (50) adapté pour attacher solidement à une surface extérieure (54) du corps (12) de la seringue (10); et
- un mécanisme à cliquet (68) relié à l'élément de base (50), où le mécanisme à cliquet étant structuré et agencé pour entrer en contact avec une tige (18) de piston du piston (14), quand le piston est positionné dans une pluralité de positions le long du corps (12) de la seringue (10), de manière pour que le contact entre le mécanisme à cliquet (68) et la tige (18) de piston permette dans chacune de cette pluralité de positions du piston (14) un mouvement distal du piston (14) par rapport au corps (12) mais résiste au mouvement proximal du piston (14) par rapport au corps ;
characterisé en ce que l'élément de base (50) comprend une composante essentiellement planare qui correspond essentiellement à la forme d'au moins une partie du corps (12) à l'extrémité proximale (28).

2. Appareil selon la revendication 1, dans lequel la tige (18) de piston comprend une forme essentiellement cylindrique, et où l'élément de base (50) comprend au moins une saillie (66) adaptée pour engager la tige (18) de piston du piston (14) quand l'élément de base (50) est attaché au corps (12), ou la saillie (66) stabilise une orientation de la tige (18) de piston par rapport au corps (12) de la seringue.

3. Appareil selon la revendication 2, dans lequel l'extrémité proximale (28) du corps (12) de seringue a une ouverture (32) adaptée pour recevoir la tige (18) de piston à travers de celle-ci, et où l'élément de base (50) comprend un passage (64) à travers de celui-ci adapté pour recevoir la tige (18) de piston à travers de celle-ci, le passage étant adapté pour s'aligner avec l'ouverture (32) à l'extrémité proximale (28) du corps (12) de seringue lorsque l'élément de base (50) est fixé au corps et dans lequel la saillie (66) s'étend dans le passage (64) de l'élément de base (50).

4. Appareil selon l'une quelconque des revendications 1 à 3, dans lequel l'élément de base (50) est conformé pour se fixer solidement à l'extrémité proximale (28) du corps (12) de telle façon qu'une forme de l'élément de base le long d'un plan perpendiculaire à un axe longitudinal du corps (12) de seringue correspond sensiblement à une forme du corps de seringue le long du plan.

5. Appareil selon l'une quelconque des revendications 1 à 4, dans lequel l'extrémité proximale du corps de seringue présente une ouverture adaptée pour recevoir une partie du plongeur à travers celle-ci, et dans lequel l'élément de base comprend un passage à travers celui-ci adapté pour recevoir la partie du plongeur à travers celle-ci, le passage étant adapté Pour s'aligner avec l'ouverture à l'extrémité proximale du corps de la seringue lorsque l'élément de base est fixé au corps.

6. Appareil selon l'une quelconque des revendications 1 à 5, comprenant en outre un clip (56) relié à l'élément de base (50), où le clip étant structuré et agencé pour se fixer solidement à une caractéristique (40) sur la surface extérieure (54) du corps (12) de seringue.

7. Appareil selon la revendication 6, dans lequel la caractéristique (40) comprend une partie d'un élément de préhension (40) s'étendant à l'écart d'un axe longitudinal du corps (12) de seringue.

8. Appareil selon l'une quelconque des revendications 1 à 7, dans lequel le mécanisme à cliquet comprend un cliquet (68) adapté pour engager une pluralité de dents (72) sur la tige (18) de piston du piston (14) quand l'élément de base (50) est attaché au corps (12).

9. Seringue (10), comprenant:
- un corps (12) ayant une extrémité proximale (28), une extrémité distale (30), et une surface extérieure (54), le corps comprenant au moins un cylindre (24), où chacun des au moins un cylindre définissant un canal de fluide (26);
- un piston (14) comportant au moins une tige (18) de piston, où chacune de l'au moins une tige (18) de piston qui peut être reçue d'une manière glissante au moins partiellement dans un des canaux de fluide (26) ; et
- un appareil selon l'une quelconque des revendications 1 à 8, où l'élément de base (50) de l'appareil est attaché solidement à la surface extérieure (54) du corps (12).

10. Seringue (10) selon la revendication 9, dans laquelle l'élément de base (50) est attaché solidement à l'extrémité proximale (28) du corps (12).

11. Seringue (10) selon la revendication 9 ou 10, dans laquelle l'élément de base (50) comprend au moins un passage (64) à travers de celui-ci, ou chacun de l'au moins un passage étant adapté pour recevoir l'une respective de l'au moins une tige de piston (18) à travers de celle-ci, et dans laquelle l'élément de base (50) comprend une pluralité de saillies (66) s'étendant dans au moins l'une des passages (64) et s'engageant dans au moins l'une des tiges (18) de piston pour stabiliser une orientation de l'au moins l'une des tiges (18) de piston par rapport au corps (12) de seringue.

12. Seringue (10) selon l'une quelconque des revendications 9 à 11, dans laquelle le corps (12) comprend au moins une caractéristique (40) sur sa surface extérieure (54), et dans laquelle l'appareil comprend un clip (56) reliée à l'élément de base (50), où le clip étant solidement fixé à la caractéristique (40).

13. Seringue (10) selon l'une quelconque des revendications 9 à 12, dans laquelle l'élément de base (50) est fixé de manière amovible à la surface extérieure (54) du corps (12).

14. Seringue (10) selon l'une quelconque des revendications 9 à 13, dans laquelle au moins l'une des tiges (18) de piston comprend une pluralité de dents (72) au moins partiellement le long de celle-ci, et dans laquelle le mécanisme à cliquet de l'appareil comprend un cliquet (68) adapté pour engager les dents (72).

15. Procédé pour modifier une seringue (10), comprenant:
- fixer solidement l'élément de base (50) d'un appareil selon l'une quelconque des revendications 1 à 8 à une surface extérieure (54) d'un corps (12) d'une seringue (10), de sorte que le mécanisme à cliquet (68) de l'appareil entre en contact avec un piston (14) de la seringue d'une manière pour permettre le mouvement distal du piston (14) par rapport au corps (12) mais de résister au mouvement proximal du piston (14) par rapport au corps.
